# EUROPÄISCHE PATENTSCHRIFT

(11) **EP 3 118 395 B1**
(45) Veröffentlichungstag und Bekanntmachung des Hinweises auf die Patenterteilung: **15.11.2017**
(21) Anmeldenummer: 16174761.3
(22) Anmeldetag: 16.06.2016
(51) Int. Cl.: E05B 1/00

(54) **VORRICHTUNG ZUR DESINFEKTION EINES MANUELL BETÄTIGBAREN GRIFFES**
DEVICE FOR DISINFECTING A MANUALLY ACTUATABLE HANDLE
DISPOSITIF DE DESINFECTION D'UNE POIGNEE A ACTIONNEMENT MANUEL

(30) Priorität: 13.07.2015 DE 102015111263
(43) Veröffentlichungstag der Anmeldung: 18.01.2017
(73) Patentinhaber: Özben, Müjdat, 31582 Nienburg/Weser (DE)
(72) Erfinder: Özben, Müjdat, 31582 Nienburg/Weser (DE)
(74) Vertreter: Scheffler, Jörg

(56) Entgegenhaltungen:
- DE-A1- 10 014 472
- DE-U1-202004 006 845
- DE-U1-202011 105 304
- US-A1- 2010 008 822
- US-A1- 2014 338 153

## Beschreibung

Die Erfindung betrifft eine Vorrichtung zur Desinfektion oder Sterilisation eines manuell betätigbaren, insbesondere drehbeweglichen Griffes vorzugsweise für Türklinken oder Türdrücker, wobei die Vorrichtung einen Behälter für ein flüssiges Desinfektionsmittel umfasst und die Vorrichtung ein an dem Griff fixiertes, flexibles Abdeckelement aufweist, welches zumindest abschnittsweise eine Grifffläche bildet, und das mittels einer Leitung mit dem Behälter verbunden ist und zumindest abschnittsweise als ein ein Reservoir für das Desinfektionsmittel bildender und dieses in Richtung der Längserstreckung des Griffes verteilender Hohlkörper mit mehreren Austrittsöffnungen ausgeführt ist, durch die das zugeführte Desinfektionsmittel auf die Grifffläche gelangt, wobei die Austrittsöffnungen für das Desinfektionsmittel im Nichtgebrauchszustand geschlossen sind oder lediglich einen sehr geringen Durchlass des Desinfektionsmittels gestatten und in einem bei der manuellen Betätigung durch eine Druckkraft belasteten Gebrauchszustand geöffnet sind.

Nach wissenschaftlichen Erkenntnissen nehmen im Krankenhaus erworbene Infektionen signifikant zu. Dieses spezifische Infektionsproblem hat in den letzten Jahren beängstigende Ausmaße angenommen, weil äußerst gefährliche Keime Resistenzen entwickelt haben, die kaum noch oder gar nicht mehr mit Antibiotika bekämpft werden können.

In Kliniken und vergleichbaren Einrichtungen werden riesige Anstrengungen unternommen, durch hygienische Maßnahmen diesem Problem Herr zu werden. Einer der neuralgischen Punkte in einer Kette von Infektionsmöglichkeiten ist die Türklinke. Hier übertragen Patienten, Ärzte, Pflegepersonal und Besucher unvermeidlich ihre Keime. Vergleichbare Probleme gibt es in Altenheime, Hotels, Praxen, bei öffentlichen Toiletten etc.; letztlich überall dort, wo fremde Personen zusammenkommen.

Insbesondere in öffentlichen Bereichen oder in größeren Geschäfts- oder Unternehmensbereichen werden Türen von vielen Personen benutzt, wobei es häufig vorkommt, dass bei einer Benutzung einer Tür an einer solchen Türklinke Keime haften bleiben, die dann bei einer nachfolgenden Benutzung von einer anderen Person aufgenommen werden können. Über Türgriffe werden schädliche Bakterien, Mikroorganismen, Keime und Viren übertragen. Es ist daher insbesondere für vielfrequentierte Türen wünschenswert, eine verlässliche Desinfektion möglich zu machen. Typisch sind beispielsweise neben den Türen angeordnete Dosierspender für Desinfektionsmittel. Solche werden aber nicht in ausreichendem Maße genutzt.

Eine gattungsgemäße Vorrichtung zur Desinfektion oder Sterilisation zur Erzielung einer antibakteriellen Wirkung wird in der Praxis bereits bei manuell betätigbaren, insbesondere drehbeweglichen Türklinken oder an dem Türblatt unbeweglichen Türdrückern eingesetzt und zählt somit zum Stand der Technik.

Aus der US 2014/338153 A1 ist eine Vorrichtung zur Desinfektion eines manuell betätigbaren Griffes bekannt, die einen Behälter für ein flüssiges Desinfektions-mittel aufweist. Der Griff ist mit einem ein Desinfektionsmittel speichernden Element in Form einer Hülle oder Hülse gebildet, die auf die Außenfläche des Griffes aufgeschoben ist. Das das Desinfektionsmittel speichernde Element besteht aus einem schwammartigen Material, das die Eigenschaft besitzt, ein Desinfektionsmittel zwischenzuspeichern und dieses bei Anwendung eines äußeren Druckes in kleinen Portionen abzugeben. Sobald also eine Bedienperson Druck auf das das Desinfektionsmittel speichernde Element ausübt, wird Desinfektionsmittel durch Poren des Elementes abgegeben und dessen Außenfläche benetzt, wodurch es zu einer Desinfektion sowohl der Außenfläche als auch der Hand der Bedienperson, die den Griff berührt, kommt.

Die DE 20 2011 105 304 U1 betrifft eine Türklinke mit einer Vorrichtung zum Desinfizierung bei Betätigung der Türklinke. Der Türgriff der Türklinke weist innen einen Hohlraum auf, der mit Desinfektionsmittel gefüllt ist. Der Hohlraum ist durch Bohrungen mit einer Umhüllung aus Schaumstoff verbunden. Wird ein Druck durch die Finger einer Hand auf den Schaumstoff ausgeübt, so wird dieser zusammengedrückt und die Desinfektionsflüssigkeit wird aus dem Schaumstoff nach außen gedrückt und kommt so mit der gesamten Hand in Berührung. Diese wird dadurch desinfiziert. Wird der Türgriff wieder losgelassen, so entsteht ein Unterdruck und die sich im Inneren des Türgriffes befindende Flüssigkeit wird in die Schaumstoffumhüllung gesaugt. Diese wird vollständig mit der Flüssigkeit durchtränkt. Dieser Vorgang wiederholt sich bei jedem Betätigen der Türklinke.

Die DE 20 2004 006 845 U1 beschreibt eine Vorrichtung zur Desinfektion eines drehbeweglichen Türgriffes, wobei die Vorrichtung einen Behälter für ein flüssiges Desinfektionsmittel aufweist. Weiterhin beschreibt auch ein an dem Griff fixiertes, flexibles Abdeckelement, welches zumindest abschnittsweise eine Grifffläche bildet und mit dem Behälter verbunden ist.

Ferner betrifft die US 2010/008822 A1 eine selbststerilisierende Kontaktoberflächenstruktur mit Öffnungen, durch die ein sterilisierendes chemisches Mittel auf die äußere Kontaktoberfläche austreten kann. Der Fluss des sterilisierenden chemischen Mittels zu der äußeren Kontaktoberfläche kann durch spezifische Ereignisse (z. B. Kontakt, bevorstehende Berührung oder Zeitablauf) ausgelöst werden. Zur Erfassung dienen mehrere Sensoren (z. B. ein Kontaktsensor, ein Annäherungssensor oder ein chemischen Sensor), die detektieren, ob ein Kontakt aufgetreten ist oder Biomaterialien auf der Außenseite vorhanden sind. Durch Steuerelemente wird die Strömung des sterilisierenden chemischen Mittels auf die Kontaktoberfläche reguliert.

Aus der EP 2 098 664 A1 ist ein Handgriff-Überzug, insbesondere für Türgriffe bekannt, wobei der Überzug vorzugsweise den Großteil der Grifffläche des Handgriffes abdeckt. Der Überzug ist fernerhin mit der Maßgabe ausgebildet, dass er im übergezogenen Zustand zumindest bereichsweise formschlüssig an dem zugeordneten Türgriff anliegt. Der Überzug hat antibakterielle Wirkung.

Bei der DE 100 14 472 A1 wird durch das Niederdrücken der Türklinke ein Pumpmechanismus betätigt, wodurch das Desinfektionsmittel aus dem Behälter, der sich auf dem Türblatt befindet, an eine Oberfläche auf dem Griffbereich geleitet.

Weiterhin ist aus der DE 10 2010 053 792 A1 eine Abdeckung von Kontaktflächen bekannt, die keimtötende Wirkstoffe enthält, die kontrolliert auf solche Oberflächen abgegeben werden, die mit den Händen in Berührung kommen, wie beispielsweise Haltegriffe oder Türklinken.

Bei der DE 10 2012 214 780 A1 wird das Desinfektionsmittel einer Düse zugeführt, der eine elektronische oder elektromechanische Steuereinrichtung für die Abgabe des Desinfektionsmittels zugeordnet ist. Aus einer Betätigung des Abdeckelementes oder der Tür kann mittels eines piezoelektrischen Elementes Energie für die Steuereinrichtung gewonnen werden.

Gemäß der DE 10 2013 014 630 A1 ist eine Dosiereinheit für das Desinfektionsmittel vorgesehen, wobei die Pumpvorrichtung eine elektrische, aus einer Batterie gespeiste Pumpe mit einer elektronischen Steuerung aufweist und durch eine Betätigung der Türklinke gesteuert wird.

Die DE 20 2004 017 284 U1 beschreibt einen Türklinkendesinfizierer, bei dem der Pumpeneffekt durch das Öffnen und Schließen der Türfläche erzeugt werden kann und bei dem das Gehäuse in den Bereich des Türdrückers oder als Aufsatzteil ausgeführt werden kann.

Ferner sind in der letzten Zeit noch Überlegungen bekannt geworden, die Betätigung der Türklinke durch ein zusätzliches Pedal an dem Türblatt zu ergänzen. So beschreibt beispielsweise die DE 10 2010 035 554 A1 einen solchen Fußtüröffner, mit einem im unteren Bereich an einer Tür angeordneten Flächenelement. Mit dem Mechanismus des Türschlosses ist das Pedal über einen Seilzug verbunden. Die Tür lässt sich aufstoßen und aufziehen. Derart primitive Überlegungen erfüllen allerdings nicht einmal die Merkmale einer Schwingtür, wie sie in der Gastronomie seit langem üblich ist, und die ebenfalls mit dem Fuß, allerdings in beide Richtungen geöffnet werden kann. Vielmehr erfordert der Fußtüröffner zum Öffnen der Tür von einer der beiden Seiten, dass der Benutzer ausreichendes Geschick besitzt, die Tür durch die Fußbetätigung zu sich heran zu ziehen. Für die Verwendung in Krankenhäusern ist ein solcher Fußtüröffner damit offensichtlich ungeeignet, weil Patienten vielfach in ihrer Mobilität eingeschränkt sind.

Als nachteilig erweist sich in der Praxis der erhebliche konstruktive Aufwand zur Installation der Vorrichtung zur Desinfektion. Insbesondere bei der Nachrüstung an vorhandenen Türanlagen sind oftmals erhebliche Eingriffe in das Türblatt sowie den Türrahmen erforderlich. Oftmals muss sogar das Türblatt vollständig ausgetauscht werden. Daher konnten sich diese an sich zweifellos sinnvollen Vorrichtungen selbst in Krankenhäusern bisher nicht durchsetzen.

Der Erfindung liegt die Aufgabe zugrunde, eine Möglichkeit zu schaffen, eine solche Vorrichtung mit geringem Aufwand auch bei vorhandenen Türanlagen problemlos einsetzen zu können. Insbesondere soll die Vorrichtung ohne wesentliche Eingriffe an der vorhandenen Tür angebracht werden können und gegebenenfalls einen vollständigen Rückbau ermöglichen.

Diese Aufgabe wird erfindungsgemäß mit einer Vorrichtung gemäß den Merkmalen des Anspruches 1 gelöst. Die weitere Ausgestaltung der Erfindung ist den Unteransprüchen zu entnehmen.

Erfindungsgemäß weist also das Abdeckelement eine flüssigkeitsdichte Innenfläche auf, die gegen den Griff anlegbar ist, und eine mit den Austrittsöffnungen ausgestattete, flexible Außenfläche, die entgegen einer elastischen Rückstellkraft verformbar ausgeführt ist, wobei die Austrittsöffnungen messerschnittartige Durchbrechungen aufweisen, die aufgrund der bei Betätigung einwirkenden Druckkraft einen kanalartigen Durchlass für das Desinfektionsmittel freigeben, sodass ausschließlich diejenigen Austrittsöffnungen aufgrund der Druckkraft geöffnet werden, die sich im Kontaktbereich mit der Hand befinden. Dadurch sind die Austrittsöffnungen für das Desinfektionsmittel im Nichtgebrauchszustand geschlossen und in einem bei der manuellen Betätigung durch eine Druckkraft belasteten Gebrauchszustand geöffnet und geben dadurch einen Durchlass für das Desinfektionsmittel frei, das unter dem Einfluss der Schwerkraft zu dem Abdeckelement fließt. Die Erfindung geht dabei von der überraschend einfachen Erkenntnis aus, dass das Desinfektionsmittel dann zielgerichtet auf die Handflächen der Benutzer übertragen werden kann, wenn die Durchlassöffnungen des den Griff bedeckenden Abdeckelementes durch den Händedruck freigegeben, also geöffnet werden. Dabei gelangt nur in den tatsächlich druckbelasteten Bereichen des Abdeckelementes das Desinfektionsmittel auf die Oberfläche. Zudem entsteht erfindungsgemäß auf der die Grifffläche bildenden Außenseite kein unerwünschter Flüssigkeitsfilm, der von den Benutzern möglicherweise als unangenehm empfunden werden kann. Vielmehr gelangt nur in den von der Handfläche bedeckten Bereichen eine solche Menge des Desinfektionsmittels auf die Grifffläche, die an der Haut des Benutzers anhaftet, sodass von einer vollständigen Übertragung ausgegangen werden kann. Bei nachlassendem Händedruck entsteht zudem ein Unterdruck, der als Saugkraft wirksam wird und überschüssige Flüssigkeitsanteile des Desinfektionsmittels in das Reservoir zurückführt. Indem das Abdeckelement eine flüssigkeitsdichte Innenfläche hat, die gegen den Griff anlegbar ist, und eine mit den Austrittsöffnungen ausgestattete, flexible Außenfläche, die entgegen einer Rückstellkraft eines zwischen der Innen- und Außenfläche eingeschlossenen elastischen Elementes verformbar ist, entsteht eine Pumpwirkung für das Desinfektionsmittel, welches zwischen der Innenfläche und der Außenfläche in dem das elastische Element einschließenden Reservoir angeordnet ist. Durch die Komprimierung des elastischen Elementes wird das darin aufgenommene Desinfektionsmittel ausgestoßen und strömt durch die Austrittsöffnungen auf die Außenfläche.

Obwohl selbstverständlich das Desinfektionsmittel in einem Behälter bevorratet werden muss, so ist dennoch mit dessen Montage nur ein geringer Aufwand verbunden. Dieser kann beispielsweise an vorhandenen Fixierpunkten des Schließmechanismus ohne zusätzliche Bohrlöcher oder durch eine rückstandsfrei entfernbare Adhäsionsverbindung fixiert werden. Denkbar ist auch ein Behälter, welcher auf dem Griff selbst angeordnet werden kann. Dabei wirkt sich vorteilhaft aus, dass die erforderlichen Mengen des Desinfektionsmittels aufgrund der äußerst zielgerichteten und damit hoch wirksamen Anwendung vergleichsweise gering sind, sodass sich selbst geringe Behälterkapazitäten in der Praxis als ausreichend erwiesen haben.

Dabei hat es sich bereits als besonders praxisgerecht erwiesen, wenn ausschließlich diejenigen Austrittsöffnungen aufgrund der Druckkraft geöffnet werden, die sich im Kontaktbereich mit der Hand befinden. Somit wird der Austritt des Desinfektionsmittels auf die relevanten Bereiche beschränkt.

Die Abdeckung könnte lediglich auf einen von der Hand erreichbaren Umfangsabschnitt des Griffes beschränkt angeordnet sein. Eine besonders vorteilhafte Ausführungsform der Erfindung wird auch dadurch realisiert, dass das Abdeckelement den Griff vollständig umfangsseitig umschließt, wobei das Abdeckelement als ein Überzug ausgeführt und vorzugsweise schlauchförmig ausgestaltet ist. Zweckmäßigerweise ist das Abdeckelement als ein schlauchförmiger Überzug mit einem offenen und einem geschlossenen Schlauchende ausgeführt. Dadurch legt sich das Abdeckelement ringförmig an den Griff an und passt sich aufgrund vorzugsweise elastischer Materialeigenschaften optimal an die Kontur des Griffes an. Eine zusätzliche Fixierung des so ausgeführten Abdeckelementes ist im Allgemeinen nicht erforderlich, weil die elastische Dehnung eine ausreichende Haftreibung erzeugt. Das Abdeckelement ist dadurch universell an nahezu beliebigen Türgriffen nachrüstbar.

Selbstverständlich kann die Vorrichtung gemäß einer Weiterbildung auch derart ausgeführt sein, dass das Abdeckelement in Umfangsrichtung des Griffes mehrere Teilbereiche mit einer unterschiedlichen Verteilung oder Größe der Austrittsöffnungen aufweist, um so das Desinfektionsmittel bedarfsgerecht auf der Grifffläche zu verteilen.

Der Behälter könnte einteilig mit dem Abdeckelement verbunden sein oder beispielsweise auch als ein integraler Bestandteil einer Rosette oder Drückerplatte des Griffes ausgeführt sein. Besonders praxisnah ist hingegen eine Ausgestaltung der Erfindung, bei welcher der Behälter durch ein Leitungselement, beispielsweise eine Schlauchleitung mit dem Abdeckelement verbunden ist. Hierdurch kann der Behälter unabhängig von dem Abdeckelement an dem Türblatt angeordnet werden, um so eine an die jeweiligen Anforderungen problemlos anpassbare Vorrichtung zu schaffen. Hierbei kann der Behälter beispielsweise auch eine äußerst geringe Aufbauhöhe haben. Ferner kann der Behälter auch in eine in die Tür einzubringende, beispielsweise stirnseitige, nutenförmige Vertiefung eingebracht werden.

Die Erfindung lässt verschiedene Ausführungsformen zu. Zur weiteren Verdeutlichung ihres Grundprinzips ist eine davon in der Zeichnung dargestellt und wird nachfolgend beschrieben.

Diese zeigt in
- Fig. 1: eine perspektivische Darstellung einer erfindungsgemäßen Vorrichtung mit einem Abdeckelement für einen Türgriff;
- Fig. 2: eine vergrößerte Schnittdarstellung des in Figur 1 gezeigten Abdeckelementes im Nichtgebrauchszustand;
- Fig. 3: das in Figur 2 gezeigte Abdeckelement in einem durch eine Druckkraft belasteten Gebrauchszustand.

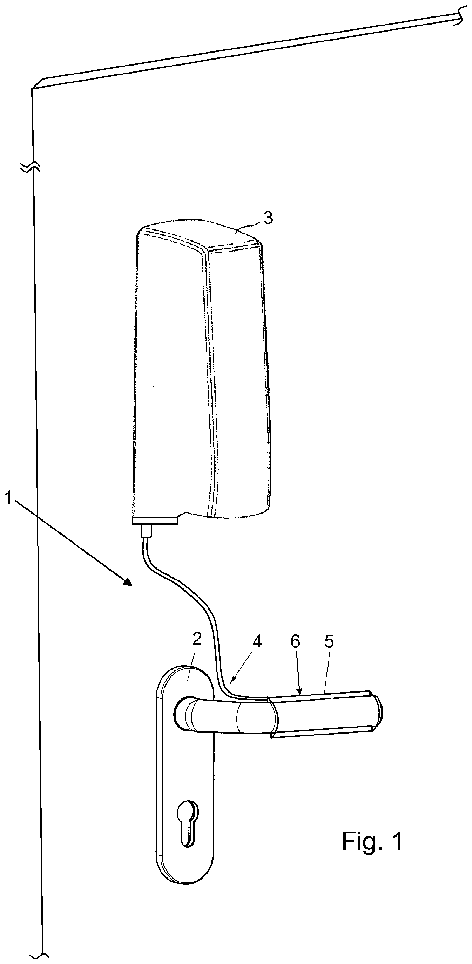

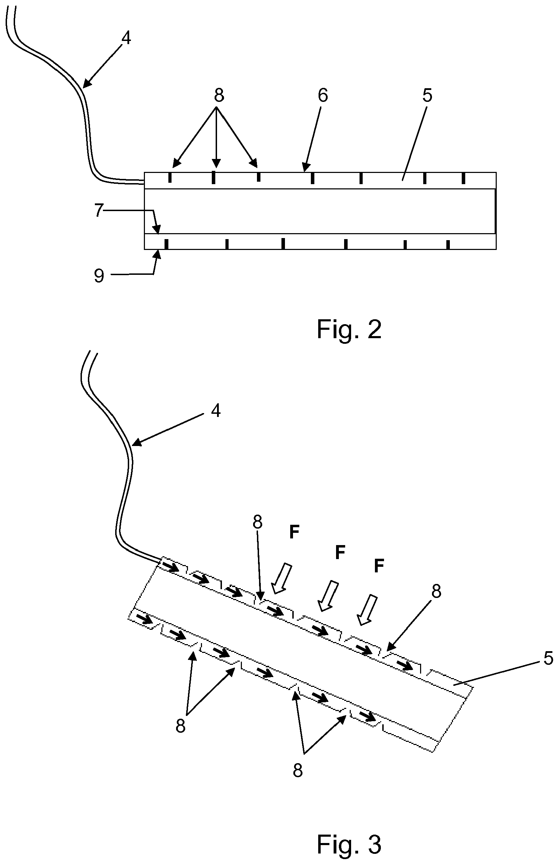

Die erfindungsgemäße Vorrichtung 1 zur Desinfektion bzw. Sterilisation eines manuell betätigbaren Türgriffes 2 wird nachstehend anhand der Figuren 1 und 2 näher erläutert. Um eine Kontaktfläche an dem Türgriff 2 mit einer ausreichenden Menge eines flüssigen Desinfektionsmittels benetzen zu können ist die Vorrichtung 1 mit einem nachfüllbaren Behälter 3 für das Desinfektionsmittel ausgestattet. Der Behälter 3 ist mittels eines als Schlauchleitung ausgeführten Leitungselementes 4 mit einem an dem Türgriff 2 fixierten, flexiblen Abdeckelement 5 verbunden, welches zumindest abschnittsweise eine Grifffläche 6 für den Benutzer bildet. Das Abdeckelement 5 ist als ein doppelwandiger Hohlkörper in Form eines elastischen Schlauchabschnittes ausgeführt und besitzt eine flüssigkeitsdichte Innenfläche 7, die gegen den Türgriff 2 anlegbar ist, und eine mit Austrittsöffnungen 8 ausgestattete, flexible Außenfläche 9, die entgegen einer elastischen Rückstellkraft verformbar ist. Dabei sind die Austrittsöffnungen 8 so ausgeführt, dass diese für das Desinfektionsmittel in einem in der Figur 2 gezeigten Nichtgebrauchszustand geschlossen sind und sich in einem bei der manuellen Betätigung durch eine Druckkraft F belasteten, in der Figur 3 gezeigten Gebrauchszustand durch den dabei wirkenden erhöhten Flüssigkeitsdruck des Desinfektionsmittels öffnen und einen V-förmigen Öffnungsspalt freigeben. Dadurch gelangt das Desinfektionsmittel aus dem Hohlkörper durch die Austrittsöffnungen 8 in den Kontaktbereich mit der Handfläche auf die Grifffläche 6 und somit auf die Haut des Benutzers. Das Abdeckelement 5 ist dabei als ein den Türgriff 2 umschließender Schlauch ausgeführt und dadurch problemlos an unterschiedlichen Türgriffen 2 fixierbar.

### BEZUGSZEICHENLISTE

- 1: Vorrichtung
- 2: Türgriff
- 3: Behälter
- 4: Leitungselement
- 5: Abdeckelement

- 6: Grifffläche
- 7: Innenfläche
- 8: Austrittsöffnung
- 9: Außenfläche
- F: Druckkraft

## Patentansprüche

1. Vorrichtung (1) zur Desinfektion oder Sterilisation eines manuell betätigbaren, insbesondere drehbeweglichen Griffes, wobei die Vorrichtung (1) einen Behälter (3) für ein flüssiges Desinfektionsmittel aufweist und wobei die Vorrichtung (1) ein an dem Griff fixiertes, flexibles Abdeckelement (5) aufweist, welches zumindest abschnittsweise eine Grifffläche (6) bildet und mit dem Behälter (3) verbunden ist, wobei das Abdeckelement (5) zumindest abschnittsweise als ein ein Reservoir für das Desinfektionsmittel bildender Hohlkörper mit mehreren Austrittsöffnungen (8) ausgeführt ist, durch die das Desinfektionsmittel auf die Grifffläche (6) gelangt, wobei die Austrittsöffnungen (8) für das Desinfektionsmittel im Nichtgebrauchszustand geschlossen sind und in einem bei der manuellen Betätigung durch eine Druckkraft (F) belasteten Gebrauchszustand geöffnet sind, **dadurch gekennzeichnet, dass** das Abdeckelement (5) eine flüssigkeitsdichte Innenfläche (7) aufweist, die gegen den Griff anlegbar ist, und eine mit den Austrittsöffnungen (8) ausgestattete, flexible Außenfläche (9), die entgegen einer elastischen Rückstellkraft verformbar ausgeführt ist und dass die Austrittsöffnungen (8) messerschnittartige Durchbrechungen aufweisen, die aufgrund der bei Betätigung einwirkenden Druckkraft (F) einen kanalartigen Durchlass für das Desinfektionsmittel freigeben, sodass ausschließlich diejenigen Austrittsöffnungen (8) aufgrund der Druckkraft (F) geöffnet werden, die sich im Kontaktbereich mit der Hand befinden.

2. Vorrichtung (1) nach Anspruch 1, **dadurch gekennzeichnet, dass** das Abdeckelement (5) den Griff vollständig umfangsseitig umschließt.

3. Vorrichtung (1) nach zumindest einem der vorhergehenden Ansprüche, **dadurch gekennzeichnet, dass** das Abdeckelement (5) zumindest abschnittsweise als ein den Griff umschließender Schlauch ausgeführt.

4. Vorrichtung (1) nach zumindest einem der vorhergehenden Ansprüche, **dadurch gekennzeichnet, dass** das Abdeckelement (5) lediglich einen Teilbereich des Griffes bildet, dem sich ein weiterer, formstabiler Teilbereich insbesondere flächenbündig anschließt.

5. Vorrichtung (1) nach zumindest einem der vorhergehenden Ansprüche, **dadurch gekennzeichnet, dass** das Abdeckelement (5) in Umfangsrichtung des Griffes mehrere Teilbereiche mit einer unterschiedlichen Verteilung und/oder Größe der Austrittsöffnungen (8) aufweist.

6. Vorrichtung (1) nach zumindest einem der vorhergehenden Ansprüche, **dadurch gekennzeichnet, dass** der Behälter (3) durch ein Leitungselement (4) mit dem Abdeckelement (5) verbunden ist.

7. Vorrichtung (1) nach zumindest einem der vorhergehenden Ansprüche, **dadurch gekennzeichnet, dass** der Behälter (3) an einem beweglichen Türblatt in einem Einschubschlitz oder an einer Außenfläche eines Türblattes angeordnet ist.

## Claims

1. Device (1) for disinfecting or sterilizing a manually actuatable handle, in particular a rotatable handle, wherein the device (1) has a container (3) for a liquid disinfecting agent, and wherein the device (1) has a flexible cover element (5) which is fixed on the handle and which at least in part forms a gripping surface (6) and is connected to the container (3), wherein the cover element (5) is configured at least in part as a hollow body forming a reservoir for the disinfecting agent, said hollow body having a plurality of outlet openings (8) through which the disinfecting agent reaches the gripping surface (6), wherein the outlet openings (8) for the disinfecting agent are closed in the not-in-use state and are opened in a use state when manually actuated and loaded by a pressure force (F), **characterized in that** the cover element (5) has a liquid-impervious inner surface (7), which can be placed against the handle, and a flexible outer surface (9), which is equipped with the outlet openings (8) and is designed to be deformable counter to an elastic restoring force, and **in that** the outlet openings (8) have notched interruptions which, on account of the pressure force (F) that arises during actuation, free a channel-like passage for the disinfecting agent, such that the only outlet openings (8) that are opened on account of the pressure force (F) are the ones located in the region of contact with the hand.

2. Device (1) according to Claim 1, **characterized in that** the cover element (5) completely encloses the circumference of the handle.

3. Device (1) according to at least one of the preceding claims, **characterized in that** the cover element (5) is designed at least in part as a tube enclosing the handle.

4. Device (1) according to at least one of the preceding claims, **characterized in that** the cover element (5) forms only a subregion of the handle, which subregion is adjoined by a further, dimensionally stable subregion, in particular in a surface-flush manner.

5. Device (1) according to at least one of the preceding claims, **characterized in that** the cover element (5) has, in the circumferential direction of the handle, a plurality of subregions with a different distribution and/or size of the outlet openings (8).

6. Device (1) according to at least one of the preceding claims, **characterized in that** the container (3) is connected to the cover element (5) by a conduit element (4).

7. Device (1) according to at least one of the preceding claims, **characterized in that** the container (3) is arranged on a movable door leaf, in an insertion slot or on an outer surface of a door leaf.

## Revendications

1. Dispositif (1) de désinfection ou de stérilisation d'une poignée à actionnement manuel, en particulier rotative, le dispositif (1) présentant un récipient (3) pour un agent de désinfection fluide, et le dispositif (1) présentant un élément de recouvrement flexible (5) fixé à la poignée qui forme au moins en partie une surface de poignée (6) et qui est relié au récipient (3), l'élément de recouvrement (5) étant réalisé au moins en partie sous forme de corps creux formant un réservoir pour l'agent de désinfection avec plusieurs ouvertures de sortie (8) à travers lesquelles l'agent de désinfection parvient sur la surface de poignée (6), les ouvertures de sortie (8) pour l'agent de désinfection étant fermées dans un état de non utilisation et étant ouvertes dans un état d'utilisation sollicité par une force de pression (F) lors de l'actionnement manuel, **caractérisé en ce que** l'élément de recouvrement (5) présente une surface intérieure (7) étanche aux liquides qui peut être appliquée contre la poignée, et une surface extérieure flexible (9) munie des ouvertures de sortie (8), qui est réalisée de manière déformable à l'encontre d'une force de rappel élastique et **en ce que** les ouvertures de sortie (8) présentent des orifices de type incision au couteau, qui, sous l'effet de la force de pression (F) agissant lors de l'actionnement, libèrent un passage de type canal pour l'agent de désinfection de telle sorte que seulement les ouvertures de sortie (8) qui se trouvent dans la zone de contact avec la main soient ouvertes sous l'effet de la force de pression (F).

2. Dispositif (1) selon la revendication 1, **caractérisé en ce que** l'élément de recouvrement (5) entoure complètement la poignée du côté de la périphérie.

3. Dispositif (1) selon au moins l'une quelconque des revendications précédentes, **caractérisé en ce que** l'élément de recouvrement (5) est réalisé au moins en partie en tant que tuyau flexible entourant la poignée.

4. Dispositif (1) selon au moins l'une quelconque des revendications précédentes, **caractérisé en ce que** l'élément de recouvrement (5) forme uniquement une région partielle de la poignée à laquelle se raccorde une région partielle supplémentaire de forme stable, en particulier en affleurement de surface.

5. Dispositif (1) selon au moins l'une quelconque des revendications précédentes, **caractérisé en ce que** l'élément de recouvrement (5) présente dans la direction périphérique de la poignée plusieurs régions partielles avec une répartition et/ou une dimension différente des ouvertures de sortie (8).

6. Dispositif (1) selon au moins l'une quelconque des revendications précédentes, **caractérisé en ce que** le récipient (3) est relié à l'élément de recouvrement (5) par un élément de conduite (4).

7. Dispositif (1) selon au moins l'une quelconque des revendications précédentes, **caractérisé en ce que** le récipient (3) est disposé sur un panneau de porte mobile dans une fente d'insertion ou sur une surface extérieure d'un panneau de porte.
